# EUROPEAN PATENT SPECIFICATION

(11) **EP 2 846 848 B1**
(45) Date of publication and mention of the grant of the patent: **07.03.2018**
(21) Application number: 13720958.1
(22) Date of filing: 08.05.2013
(51) Int. Cl.: A61L 26/00, A61K 9/00

(54) **OPHTHALMIC VISCOELASTIC DEVICE**
VISKOELASTISCHE OPHTHALMISCHE VORRICHTUNG
DISPOSITIF OPHTALMIQUE VISCOÉLASTIQUE

(30) Priority: 10.05.2012 GB 201208625
(43) Date of publication of application: 18.03.2015
(73) Proprietor: Carl Zeiss Meditec AG, 07745 Jena (DE)
(72) Inventor: WOLFSTEIN, André, 13507 Berlin (DE); RODDEN, Gillian, Edinburgh EH14 2LG (GB); NACHBAUR, Jürgen, 13509 Berlin (DE); MOISSONNIER, Claude, 53175 Bonn (DE); BERNARD, Pascal, F-17137 Nieul sur Mer (FR); LOREC, Gildas, F-17290 Ciré d'Aunis (FR); BIELEFELDT, Nicole, 10437 Berlin (DE); RATHERT, Brian, 10115 Berlin (DE); NACHBAUR, Lidia, 13509 Berlin (DE); GERLACH, Mario, 16540 Hohen Neuendorf (DE); RENNIE, Alistair, Bothwell Glasgow G71 BRY (GB)
(74) Representative: Hofstetter, Schurack & Partner
(86) International application number: PCT/EP2013/059546
(87) International publication number: WO 2013/167632

(56) References cited:
- WO-A1-86/02548
- WO-A1-88/09663
- US-A1- 2005 215 516
- US-A1- 2006 073 184

## Description

### Field of the Invention

The invention relates to an ophthalmic viscoelastic device comprising at least one viscoelastic polymer. The invention further relates to a method for producing an ophthalmic viscoelastic device comprising at least one viscoelastic polymer.

### Prior Art

Cataract is a common disease especially in elderly people, where the crystalline lens gradually becomes less transparent. This opacity of the natural lens leads to the loss of visual acuity. To restore vision, cataract surgery has to be performed. The standard method to remove the opaque nucleus of a lens to create a capsular bag for insertion of an artificial intraocular lens (IOL) is the so-called phacoemulsification by a device which generates ultrasound oscillations. Especially the endothelial layer of orderly arranged cuboidal cells, which are located on the Descemet membrane at the inner side of the cornea, can be affected by this iatrogenic procedure.

The endothelial cell layer maintains corneal clarity by mediating a net flux of ions from the stroma to the aqueous humor which keeps the stroma in a relatively dehydrated state. The density of these cells is for an elderly cataract patient app. 2600 cells/mm². Corneal endothelial cells are not able to divide and if their density decreases down to approx. 500 cells/mm² there is a risk of corneal edema because the dehydrated state of the stroma and therefore the clarity cannot be maintained any more. It is one of the most serious complications which were noted frequently in early phacoemulsification technology. The delicate endothelial cell layer may be damaged e.g. due to mechanical trauma by instruments, impact of lens fragments, or by the biochemical and mechanical effects of the irrigation solution.

Immediately before phacoemulsification, the anterior chamber is usually filled with a so-called ophthalmic viscoelastic device (OVD) in order to protect the endothelium. The viscoelastic OVD is used as a surgical aid to protect intraocular tissues (for example the corneal endothelium during phacoemulsification), as a space maintainer (for example to maintain the anterior chamber of the eye) and to facilitate intraocular maneuvers, for example to make a controlled capsulorhexis.

Ophthalmic viscoelastic devices usually are water-based solutions containing viscoelastic polymers like hyaluronic acid, hydroxypropylmethylcellulose, chondroitin sulfate or mixtures thereof. The viscoelastic composition might differentiate in the molecular weight of the polysaccharide dissolved in the solution, in the concentration of the polysaccharide and in the viscosity of the solution. OVDs are generally well-tolerated following brief exposure to intraocular tissues.

However, a further side effect of the phacoemulsification process is the generation of free radicals which may effect especially the endothelial cells located on the inner side of the cornea. This monolayer of cells is essential to maintain the clarity of the cornea. The free radicals, in particular hydroxyl radicals, are the result of ultrasonic oscillations that induce acoustic cavitations which lead to dissociation of water vapor. It has been proposed that these free radicals are a significant source of endothelial damage after cataract extraction by inducing apoptosis.

The addition of a scavenger to OVDs can reduce the concentration of these radicals and therefore help in protecting the ocular tissue. The additional use of scavengers helps to buffer the amount of oxygen-free radicals and therefore increases the protection performance of OVDs besides their viscoelastic properties, suitable pH values and suitable osmolality to reduce the loss of endothelial cells during cataract surgery.

Document WO 88/09663 A1 discloses a viscoelastic vitreous substitute for blocking ultraviolet light during ophthalmic surgery which comprises approximately 2% of a hydroxy-substituted cellulose derivative in a physiologic balanced salt solution covalently bonded to approximately 0.2% of a UV-absorbant molecule.

Document WO 86/02548 A1 discloses a composition to be used in ophthalmology and containing an aqueous solution of a high molecular polymer to which has been added a polymeric dye having a molecular weight higher than 10,000.

Document US 2005/0215516 A1 discloses a viscoelastic composition comprising an aqueous solution of a viscoelastic polymer. The viscoelastic composition contains tris[hydroxymethyl]aminomethane and a polyol like sorbitol as scavengers. However, these scavengers can diffuse out of the OVD into the ocular tissue or body fluids and may cause unwanted side effects. Diffusion into body fluids may for example cause unwanted local or systemic pharmacological effects caused by binding to a receptor target. Further problems arise if hydrophobic scavengers with relatively low water solubility are used as they may precipitate out of the solution and accumulate in the ocular tissue.

### Detailed Description of the Invention

It is the object of the present invention to improve an ophthalmic viscoelastic device of the initially mentioned type such that it is simpler and safer in application. A further object of the invention is to provide a method for producing such an ophthalmic viscoelastic device.

According to the invention, these objects are achieved by an ophthalmic viscoelastic device having the features of claim 1 as well as by a method for producing an ophthalmic viscoelastic device having the features of claim 10. Advantageous developments of the invention are specified in the respective dependent claims, wherein advantageous developments of the ophthalmic viscoelastic device are to be regarded as advantageous developments of the method and vice versa.

In an ophthalmic viscoelastic device (OVD) according to a first aspect of the invention, comprising at least one viscoelastic polymer, it is provided that the at least one viscoelastic polymer is covalently bound to at least one phenolic compound. Therein, within the scope of the invention, chemical compounds are to be understood by the term phenolic compound belonging to the class of chemical compounds comprising at least one hydroxyl group (-OH) bonded or linked directly to an aromatic hydrocarbon group according to the general formula 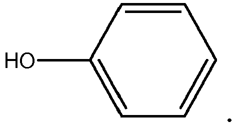 The simplest of the class is phenol which is also called carbolic acid (C₆H₅OH).

Covalent binding or linking of a phenolic compound to the viscoelastic polymer offers various advantages. First, phenolic compounds exhibit high antioxidant activity and scavenge free radicals, thereby diminishing the concentrations of reactive oxygen species in the patient's eye. Phenolic compounds are able to form stable radicals because of the delocalization of the impaired electron into the aromatic electron system. The amount of free radicals produced during phacoemulsification can be identified by electron spin resonance (ESR) spectroscopy. An in vitro set up for testing this radical scavenging effect has been described by Cameron et al. (Identification of free radicals produced during phacoemulsification, JCRC 2001; 27: 463-470). The amount and concentration of the phenolic compound in the OVD can then be adjusted accordingly. Further, the fixation of the phenolic compound to an OVD will restrict the radical scavenging activity to the area where the free radicals are generated during phacoemulsification. Still further, by covalent binding of the phenolic compound to the at least one viscoelastic polymer, it is reliably excluded that the scavenger diffuses into adjoining tissue during use of the OVD. Also, phenolic scavenger compounds with low water solubility can be used because aggregation or precipitation are prevented by the attachment to the viscoelastic polymer. In simplest configuration, the OVD is composed of a solution of a single viscoelastic polymer, to which a phenolic compound is covalently bound. Alternatively, the ophthalmic viscoelastic device, which could also be denominated as an ophthalmologic composition, can include plural different viscoelastic polymers and/or further additives. Independently thereof, it can also be provided that two or more different phenolic compounds are covalently bound to a viscoelastic polymer. According to the invention, it is provided that the at least one viscoelastic polymer is covalently bound to at least one dye. Therein, within the scope of the invention, chemical compounds are to be understood by dye, which include at least one chromophore molecule structure, which absorbs light in the wavelength range visible to the human between about 380 nm and about 800 nm and preferably does not exhibit any fluorescence or phosphorescence. By the covalent bond of the dye to the at least one viscoelastic polysaccharide, it is reliably excluded that the dye diffuses into adjoining tissue and undesirably stains it during use of the ophthalmic viscoelastic device or the ophthalmologic composition. Moreover, unlike fluorescent dyes such as fluorescein, rhodamine or the like, it is not required to irradiate the ophthalmic viscoelastic device with UV light to visualize it. This allows a substantially simpler handling of the ophthalmic viscoelastic device. In addition, there is a priori no risk that the concerned tissue is damaged by the irradiation with high-energy UV light. Further, a user - for example a surgeon - can even recognize minor traces without additional auxiliary means such as UV lamps or the like due to the integral coloration of the ophthalmic viscoelastic device, whereby the use of the ophthalmic viscoelastic device according to the invention in addition becomes substantially simpler and safer - for example within the scope of eye surgeries. In simplest configuration, the ophthalmic viscoelastic device is composed of one viscoelastic polymer, to which one specific dye is covalently bound. Also, it can be provided that the ophthalmic viscoelastic device is composed of two or more different viscoelastic polymers, wherein one specific dye is covalently bound to one or more of the polymers. Independently thereof, it can also be provided that two or more different dyes are covalently bound to a viscoelastic polymer in order to provide a specific color.

In an advantageous development of the invention, it is provided that the at least one phenolic compound is bound directly and/or via a spacer to the viscoelastic polymer. Hereby, the phenolic compound can be particularly simply adapted to the present reactive groups of the concerned polymer and be covalently bound to it. The use of a spacer is for example advantageous if the reactive group of the phenolic compound cannot be bound to a corresponding reactive group of the polymer or only within the scope of multi-stage reactions. Furthermore, the use of a spacer is advantageous if the scavenger behavior of the phenolic compound would otherwise be affected in undesired manner by the covalent binding to the polymer. Finally, by the use of a spacer the viscoelastic properties of the polymer can be influenced as needed.

In a further advantageous development of the invention, it is provided that the viscoelastic polymer comprises a polysaccharide. Hereby the ophthalmic viscoelastic device has a particularly high biological compatibility. Preferably, the polysaccharide is a glycosaminoglycan.

In a further advantageous development of the invention, it is provided that the polysaccharide is cellulose, a cellulose ether with methyl and/or ethyl and/or propyl groups, in particular hydroxypropyl methylcellulose, hydroxyethyl methylcellulose and/or methylcellulose, a glycosaminoglycan, in particular hyaluronic acid, chondroitin sulphate, dermatan sulphate, heparin, heparan sulphate, keratan sulphate, alginic acid, polymannuronic acid, polyguluronic acid, polyglucuronic acid, amylose, amylopectin, callose, chitosan, polygalactomannan, dextran, xanthan and/or a mixture thereof. Hereby, in particular the viscoelastic properties of the OVD can be adapted to the respective purpose of employment and use in optimum manner. Therein, basically, it can also be provided that the OVD includes two or more polysaccharides of the same type, which may only differ with regard to the kind and/or molecular proportion of covalently bound phenolic compound.

In a further advantageous development of the invention, it is provided that the dye comprises a phenolic compound and/or a reactive dye from the group of modified and/or unmodified aminoanthracenedione and/or of modified and/or unmodified nitrophenyldiazenylbenzenamine. In other words it is envisaged that the phenolic compound itself is a dye. Hereby the phenolic compound is advantageous both to scavenge radicals as well as to be used as dye, whereby the ophthalmic viscoelastic device on the one hand can be produced at particularly low cost and on the other hand offers particularly safe and easy handling. Alternatively or additionally it is provided that the at least one viscoelastic polymer is obtainable by a reaction of the polymer with at least one reactive dye from the group of modified and/or unmodified aminoanthracenedione and/or modified and/or unmodified nitrophenyldiazenylbenzenamine. The use of an aminoanthracenedione or of an aminoanthracenedione derivative and/or of a nitrophenyldiazenylbenzenamine or of a nitrophenyldiazenylbenzenamine derivative as a dye, which is covalently bound to the polymer, therein offers the advantage that the color of the viscoelastic polymer and thereby of the ophthalmic viscoelastic device is specifically adjustable nearly in the entire visible wavelength range. Furthermore, these two dyes or dye groups are characterized by a great fastness to washing and light. Thus, the ophthalmic viscoelastic device is particularly simple and safe to handle and additionally has high storage stability.

Therein, the at least one polymer may be obtainable by reaction with at least one reactive dye of the general formula (I), 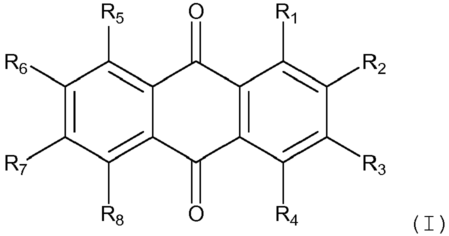 wherein in the general formula (I) at least one of the substituents R₁ to R₈ is an amino group, and the remaining substituents R₁ to R₈, which are different from the amino group are selected from hydrogen, halogen, C₁-C₄-alkyl, C₁-C₄-alkoxy, C₁-C₄-alkoxy-C₁-C₄-alkyl, C₁-C₄-alkyl-C₁-C₄-alkoxy, C₁-C₄-alkoxy-C₁-C₄-alkoxy, aryloxy, acetamide, propionamide, butyramide, isobutyramide, sulphonate, (aminophenyl)-N-alkylacetamide, (aminophenylsulphonyl)alkyl sulphate, alkylbenzenesulphonamide, tri(alkyl)benzenamine, hydroxy, (alkylsulphonyl)benzenamine, and (alkenylsulphonyl)benzenamine, and/or wherein at least two adjacent substituents Rₓ, Rₓ₊₁ with x=1 to 3 and/or 5 to 7 form a 3-, 4-, 5-, 6- or 7-membered homocyclic or heterocyclic radical, wherein said cyclic radical can be unsaturated or aromatic. Therein, all of the stereoisomers, racemic mixtures and position isomers are to be considered as included. With the aid of a dye of the general formula (I), the color properties of the polysaccharide covalently bound to it can be varied within wide limits, wherein combinations of different dyes of the general formula (I) can basically also be used to achieve a specific coloration. By at least one of the radicals R₁ to R₈ being an amino group, the dye can additionally be coupled to a plurality of functional groups in chemically particularly simple manner. Therefore, different viscoelastic polymers with correspondingly different functional groups can also quickly and simply be coupled to the dye of the general formula (I) without the requirement of costly multi-stage reactions, thereby resulting significant cost advantages. All reactive dyes of the general formula (I) exhibit a high extinction coefficient in the visible range of the light spectrum.

Further it may be provided that the at least one polysaccharide is obtainable through reaction with at least one reactive dye of the general formula (II), 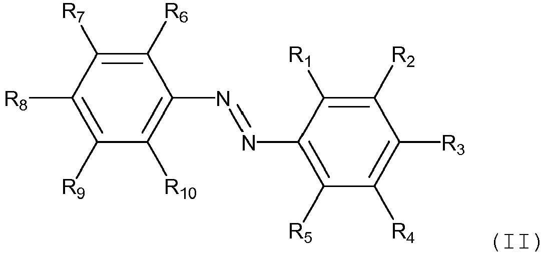 wherein in the general formula (II) at least one of the substituents R₁ to R₅ is an amino group and at least one of the substituents R₆ to R₁₀ is a nitro group, and wherein the remaining radicals R₁ to R₁₀, which are different from the amino group and the nitro group are selected from hydrogen, halogen, C₁-C₄-alkyl, C₁-C₄-alkoxy, C₁-C₄-alkoxy-C₁-C₄-alkyl, C₁-C₄-alkyl-C₁-C₄-alkoxy, C₁-C₄-alkoxy-C₁-C₄-alkoxy, aryloxy, acetamide, propionamide, butyramide, isobutyramide, sulphonate, (aminophenyl)-N-alkylacetamide, (aminophenylsulphonyl)alkyl sulphate, alkylbenzenesulphonamide, tri(alkyl)benzenamine, hydroxy, (alkylsulphonyl)benzenamine, and (alkenylsulphonyl)benzenamine, and/or wherein at least two adjacent substituents R_{y}, R_{y+1} with y=1 to 4 and/or 6 to 9 form the cyclic radical 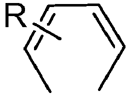 with: R=hydrogen, halogen, amino, hydroxyl, nitro, C₁-C₄-alkyl, C₁-C₄-alkoxy, C₁-C₄-alkoxy-C₁-C₄-alkyl, C₁-C₄-alkyl-C₁-C₄-alkoxy, and/or C₁-C₄-alkoxy-C₁-C₄-alkoxy. Therein, all of the stereoisomers, racemic mixtures and position isomers are to be considered as included. With the aid of a dye of the general formula (II), the color properties of the viscoelastic polymer covalently bound to it can also be varied within wide limits, wherein combinations of different dyes of the general formula (II) can basically also be used to achieve a specific coloration. By at least one of the radicals R₁ to R₅ being an amino group and at least one of the radicals R₆ to R₁₀ being a nitro group, a particularly high extinction coefficient in the visible range of the light spectrum results. With the aid of the at least one amino group, the dye can additionally be covalently bound to a plurality of functional groups in chemically particularly simple manner. Therefore, different viscoelastic polymers with correspondingly different functional groups can also quickly and simply be coupled to the dye of the general formula (II) without the requirement of costly multi-stage reactions, thereby producing significant cost advantages.

In a further advantageous development of the invention, it is provided that the at least one phenolic compound is selected from a group of modified and/or unmodified hydroxycinnamic acids, phenylpropenes, cumarins, hydroxy-cumarines, isocoumarins, chromones, hydroxybenzoic acids, in particular salicylic acid and acetylsalicylic acid, tocopheroles, tocotrienols, propofol, phenolic acids, phenolic aldehydes, N-(2,3-dihydro-7-hydroxy-2,2,4,6-tetramethyl-1H-inden-1-yl)-4-(3-methoxyphenyl)-1-piperazineacetamide, butylhydroxyanisole, butylhydroxy-toluene, galvinoxyl, benzoquinones, acetophenones, tyrosine derivatives, phenylacetic acids, naphthoquinones, xanthonoids, stilbenoids, in particular resveratrol, anthraquinones, flavonols, dihydroflavonols, tannins, pseudo tannins, anthocyanins, anthocyanidins, flavanol monomers, in particular catechins, flavanol polymers, in particular proanthocyanidins, flavanones, flavones, chalconoids, isoflavonoids, neoflavonoids, lignans, neolignans, biflavonoids, catechol melanins, flavolans, theaflavins, and thearubigins. In this way the radical-scavenging properties, the viscoelastic properties and last but not least also the coloration of the ophthalmic viscoelastic device can be optimally adjusted to the respective purpose of the application.

By modified and/or unmodified hydroxycinnamic acids are to be understood for instance 3-caffeoyl quinic acid, 3-p-caffeoyl quinic acid, 4-caffeoyl quinic acid, 5-caffeoyl quinic acid, 3-feruloyl quinic acid, p-cumaroylglucose, feruloylglucose, caffeic acid-4-O-glucoside, p-coumaric acid-O-glucoside, and feruloylic acid-O-glucoside.

The term modified and/or unmodified cumarins or hydroxy-cumarines refers for instance to cumarin, umbelliferone, herniarin, esculetin, scopoletin, and fraxetin.

By modified and/or unmodified phenolic acids and hydroxybenzoic acids for instance phenolic acid, hydroxycinnamic acid, coumaric acid, ferulic acid, caffeic acid, sinapic acid, gallic acid, salicylic acid, acetylsalicylic acid, 4-hydroxy benzoic acid, 2,3-dihydroxybenzoic acid, 3,4-dihydroxybenzoic acid, 3,4,5-trihydroxybenzoic acid, 3-methoxy-4-hydroxybenzoic acid and ellagic acid are to be understood.

Tocopheroles comprise in particular alpha-Tocopherol, beta-Tocopherol, gamma-Tocopherol, and delta-Tocopherol. Tocopherols and tocotrienols are fat-soluble antioxidants. By way of covelantly binding one or more of these compounds to the visoelastic polymer, the OVD can also be used advantageously in an aqueous solution.

Suitable stilbenoids exhibiting particulary good scavenger properties comprise the aglycones piceatannole, pinosylvine, pterostilbene, and resveratrol.

Tannins are polyphenolic compounds from plants and have molecular weights ranging from 500 to over 3,000 (gallic acid esters) and up to 20,000 (proanthocyanidins) g/mol. There are three major classes of tannins. The base unit of the first class consists of gallic acid, the base unit of the second class consists of flavone, and the base unit of the third class consists of phloroglucinol. Pseudo tannins are low molecular weight compounds associated with other compounds. Tannins and pseudo tannins exhibit good antioxidant properties.

The term modified and/or unmodified flavanol monomers and flavanol polymers refers in particular to catechins like catechin, epicatechin, gallocatechin, epigallocatechin, proanthocyanidins, and prodephinidine. Proanthocyanidins and prodephinidines are colorless pre-stages of anthocyanidins. In a sour environment carbocations can be generated from proanthocyanidins and prodephinidines. The carbocations are for instance formed in the presence of oxygen to form colored anthocyanidins or anthocyanidines.

Anthocyanins and anthocyanidines, which are flavylium cation derivatives of anthocyanins, may appear red, purple, blue, or yellow according to the pH. This means, that by covalently coupling the viscoelastic polymer with a phenolic compound from the group of anthocyanins and/or anthocyanidines the ophthalmic viscoelastic device can be provided with scavenger properties and with pH-dependent color properties. Therefore, the ophthalmic viscoelastic device is easy to manage and can advantageously be used to easily detect or indicate normal or abnormal pH-values in the ocular tissue. Anthocyanidins comprise e.g. aurantinidin, cyaniding, delphinidin, europinidin, luteolinidin, pelargonidin, malvidin, peonidin, petunidin, and rosinidin.

Flavanones and flavones are a class of flavonoids based on the backbone of 2-phenylchromen-4-one (2-phenyl-1-benzopyran-4-one) and exhibit particularly good scavenger properties. Still further, flavones have the additional advantage of being colored compounds. Flavones that can be used in connection with the present invention include for example apigenin, acacetin, luteolin, chrysin, chrysoeriol, diosmetin, tectochrysin, scutellarein, eupatorin, genkwanin, sinensetin, and wogonin. Synthetic flavones include for example 4'-amino-6-hydroxyflavone, diosmin and flavoxate.

Suitable theaflavines for use in the present invention include e.g. theaflavin-3-gallate, theaflavin-3'-gallate, and theaflavin-3-3'-digallate. Still further thearubigins may be used which are polymeric polyphenols that are usually formed during the enzymatic oxidation of tea leaves. Thearubigins are usually red in color.

In a further advantageous development of the invention, it is provided that the at least one phenolic compound is glycosylated. Glycosylation denotes a reaction in which a carbohydrate is attached to a hydroxyl or other functional group of the phenolic compound, which acts as a glycosyl acceptor. Glycosylation may serve a variety of structural and functional roles and in particular may be used to influence the viscoelastic properties and the biological tolerance of the viscoelastic polymer. Examples for glycosylated phenolic compounds are anthocyanins like Cy-3-gal, Cy-3-ara, Cy-7-ara, Cy-3-gal, Cy-3-glc, Cy-3-glc, Cy-3-rut, Peo-3-glc, Peo-3-rut, Cy-3-sop, Cy-3-glc-rut, Pg-3-glc, Pg-3-gal, Cy-3-glc, Pet-3-gly, Del-3-glc, Del-3-gal, Mv-3-glc, Cy-3-glc-sop, Cy-3-xyl-rut, Cy-3-sam, Mv-3,5-diglc, Mv-3-p-cumaroylglc-5-glc, Mv-3-p-coffeoylglc-5-glc, Peo-3-p-cumaroylglc-5-glc, or Del-3-glc-glc with Cy = cyaniding, Del = delphinidin, Mv = malvidin, Peo = peonidin, Pet = petunidin, Pg = pelargonidin and ara = arabinoside, gal = galactoside, glc = glucoside, gly = glycoside, rut = rutinoside, sam = sam-bubioside, sop = sophoroside, xyl = xyloside, glc-rut = glucosyl-rutinoside etc.

Further advantages arise by the zero-shear viscosity of the ophthalmic viscoelastic device being between 20,000 and 8,000,000 mPa.s, preferably between 50,000 and 500,000 mPa.s. Hereby, the ophthalmic viscoelastic device has a particularly good flow behavior and is correspondingly well to handle in typical applications. Preferably the ophthalmic viscoelastic device is an aqueous solution of the viscoelastic polymer, which is covalently bound to the at least one phenolic compound.

In a further advantageous development of the invention, it is provided that the ophthalmic viscoelastic device is a water-based ophthalmologic solution to protect intraocular tissues in eye surgery, wherein the solution contains the viscoelastic polymer, to which the at least one phenolic compound is covalently bound. Due to the integral, non-washable and simply adjustable scavenger properties of the ophthalmic viscoelastic device, the OVD comprising the aqueous solution of the viscoelastic polymer can be introduced into the eye in a particularly simple and controlled manner without the risk of washout of the phenolic compound or of adversely affecting the surrounding tissue being involved. The entire ophthalmic viscoelastic device can afterwards be removed from the eye without residue in correspondingly simple and safe manner for example after a cataract surgery. It may be provided that the ophthalmic viscoelastic device is prepared for use in ophthalmology, in particular for use in a phacoemulsification method.

A second aspect of the invention relates to a method for producing an ophthalmic viscoelastic device comprising the steps of covalently bonding the at least one viscoelastic polymer to at least one phenolic compound, covalently bonding the at least one viscoelastic polymer to at least one dye, and using the at least one viscoelastic polymer as an ingredient of the ophthalmic viscoelastic device. By the covalent binding of the at least one phenolic compound to the at least one viscoelastic polymer, it is reliably excluded that the phenolic compound, which acts as a scavenger for free radicals, diffuses into adjoining tissue and adversely affects it during the use of the ophthalmologic composition. This allows a substantially simpler handling of the ophthalmologic composition. In addition, the concerned tissue is reliably protected against free radicals. In simplest configuration, the ophthalmic viscoelastic device, which may also be called an ophthalmologic composition, is produced with a viscoelastic polymer, to which a single type of a phenolic compound is covalently bound. Alternatively, it can be provided that plural different viscoelastic polymers and/or further additives are used to produce the ophthalmic viscoelastic device. Independently thereof, it can also be provided that two or more different phenolic compounds are covalently bound to a viscoelastic polymer, whereupon the viscoelastic polysaccharide - optionally with further additives - is used for producing the ophthalmic viscoelastic device. The respective suitable reaction type for covalently binding the phenolic compounds to the viscoelastic polymer depends on the functional groups present in each case. It is further provided that the at least one viscoelastic polymer is covalently bound to at least one dye. Therein, within the scope of the invention, chemical compounds are to be understood by dye, which include at least one chromophore molecule structure, which absorbs light in the wavelength range visible to the human between about 380 nm and about 800 nm and preferably does not exhibit any fluorescence or phosphorescence. By the covalent bonding of the dye to the at least one viscoelastic polysaccharide, it is reliably excluded that the dye diffuses into adjoining tissue and undesirably stains it during use of the ophthalmic viscoelastic device or the ophthalmologic composition. Moreover, unlike fluorescent dyes such as fluorescein, rhodamine or the like, it is not required to irradiate the ophthalmic viscoelastic device with UV light to visualize it. This allows a substantially simpler handling of the ophthalmic viscoelastic device. In addition, there is a priori no risk that the concerned tissue is damaged by the irradiation with high-energy UV light. Further, a user - for example a surgeon - can even recognize minor traces without additional auxiliary means such as UV lamps or the like due to the integral coloration of the ophthalmic viscoelastic device, whereby the use of the ophthalmic viscoelastic device according to the invention in addition becomes substantially simpler and safer - for example within the scope of eye surgeries. In simplest configuration, the ophthalmic viscoelastic device is composed of one viscoelastic polymer, to which one specific dye is covalently bound. Also, it can be provided that the ophthalmic viscoelastic device is composed of two or more different viscoelastic polymers, wherein one specific dye is covalently bound to one or more of the polymers. Independently thereof, it can also be provided that two or more different dyes are covalently bound to a viscoelastic polymer in order to provide a specific color

Conjugates between the viscoelastic polymer and a phenolic compound comprising at least one free amino group can be obtained by the reaction of the CNBr-activated polymer with this phenolic compound. Further, it is provided that a phenolic compound with an amino group is used and covalently bound to the polysaccharide by way of an Ugi reaction. The Ugi reaction is a multi-component reaction involving a ketone or aldehyde, an amine, an isocyanide and a carboxylic acid. The products of the Ugi reaction are bis-amides. The Ugi reaction is an uncatalyzed reaction but is usually completed within minutes after adding the isocyanide. The main advantages of using an Ugi reaction are the inherent high atom economy as only a molecule of water is lost and the high product yields. Accordingly, the dye including the amino group can be bound quickly, simply and with high yields to polymers comprising functional keto groups, aldehyde groups and/or carboxylic acid groups. An example for a suitable viscoelastic polymer is hyaluronic acid. Hyaluronic acid is a polysaccharide with repeating disaccharide units of glucuronosyl-β-1,3-N-acetylglucosamine linked by β-1,4-glycoside (glycosidic) bonds and has the general formula

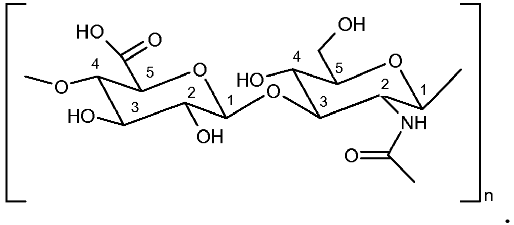

Still further, the so-called Williamson ether synthesis can be used, which is an organic reaction, forming an ether from an organohalide and an alcohol involving the reaction of an phenolate ion with a primary halide of the viscoelastic polymer. Still further, the coupling reaction does not necessarily have to take place in the same medium. The coupling reaction may also take place at the interface between two phases of immiscible solvents containing the phenolic compound in one solvent phase and the viscoelastic polymer in the other solvent phase. This is known as interfacial reaction in a two-phase system. However, the method of the second inventive aspect generally is not limited to a specific synthetic pathway. Further advantages rendered can be gathered from the explanations given as to the first aspect of the invention, wherein advantageous embodiments of the first aspect of the invention are to be regarded as advantageous embodiments of the second aspect of the invention and vice versa.

In an advantageous development of the invention, it is provided that for producing the ophthalmic viscoelastic device the viscoelastic polymer, which is covalently bound to the at least one phenolic compound, and/or a physiologically acceptable salt thereof is dissolved and/or dispersed in an amount of between 0.05 percent by weight and 5 percent by weight possibly together with a buffer system and/or with further agents and/or excipients in a protic solvent, in particular in water. Within the scope of the invention, by an amount of between 0.05 percent by weight and 5 percent by weight, in particular amounts of 0.05 %, 0.15 %, 0.25 %, 0.35 %, 0.45 %, 0.55 %, 0.65 %, 0.75 %, 0.85 %, 0.95 %, 1.05 %, 1.15 %, 1.25 %, 1.35 %, 1.45 %, 1.55 %, 1.65 %, 1.75 %, 1.85 %, 1.95 %, 2.05 %, 2.15 %, 2.25 %, 2.35 %, 2.45 %, 2.55 %, 2.65 %, 2.75 %, 2.85 %, 2.95 %, 3.05 %, 3.15 %, 3.25 %, 3.35 %, 3.45 %, 3.55 %, 3.65 %, 3.75 %, 3.85 %, 3.95 %, 4.05 %, 4.15 %, 4.25 %, 4.35 %, 4.45 %, 4.55 %, 4.65 %, 4.75 %, 4.85 %, 4.95 %, and 5.00 % as well as corresponding intermediate values are to be understood. Hereby, the property profile of the ophthalmologic composition can be adapted to different purposes of application in an optimum manner.

In a further advantageous development of the invention, it is provided that in order to bond the at least one phenolic compound, the viscoelastic polymer and/or the at least one phenolic compound is functionalized and covalently bound via the added functional group. In other words, first, a functional group is introduced into the viscoelastic polymer, which is subsequently reacted with the at least one phenolic compound, in order to covalently bind the at least one phenolic compound to the polymer. In this manner, the at least one phenolic compound can be simply bound to different polymers with correspondingly specific functional groups largely independent of its precisely present reactive group. Alternatively or additionally, it can be provided that the at least one phenolic compound is first functionalized and subsequently covalently bound to the viscoelastic polysaccharide via its newly created functional group by means of a desired type of reaction.

In a further advantageous development of the invention, it is provided that the at least one phenolic compound is covalently bound to a carboxylic group and/or to a primary hydroxyl group of the viscoelastic polymer. The easiest way to covalently bind the phenolic compound to the viscoelastic polymer comprises the use of an esterification method. For example, the phenolic hydroxyl groups of the phenolic compound - e.g. a hydroxyflavone - may bind by esterification to carboxyl-groups of the viscoelastic polymer, e.g. to hyaluronic acid, via acid chlorides or acid anhydrates in the presence of potassium carbonate or pyridine according to the Schotten-Baumann reaction. An ester preparation may comprise e.g. the use of hyaluronic acid and alpha-tocopherol with vitamin A acid under mild conditions with trifluoroacetic acid anhydride as catalytically active component. Still further, the phenolic compound can be bound to carboxyl-groups of the viscoelastic polymer by the mild Steglich esterification using dicydohexylcarbodiimid (DCC) and 4-(dimethylamino)-pyridin (DMAP) as catalyst.

In a further advantageous development of the invention, it is provided that a viscoelastic polymer having a molecular weight of between 500,000 u and 5,000,000 u, preferably between 800,000 u and 2,500,000 u, is covalently bound to the at least one phenolic compound. Hereby too, a particularly good flow behavior and a correspondingly good manageability of the ophthalmic viscoelastic device is achieved. In addition, plural phenolic molecules can be covalently coupled to a viscoelastic polymer molecule without this resulting in substantial variations of the viscoelastic properties of this polymer.

Further features of the invention appear from the claims as well as based on the following embodiments.

### Preferred Embodiments of the Invention

The present invention generally provides an ophthalmic viscoelastic device (OVD), comprising at least one viscoelastic polymer that is covalently bound to a compound that is adapted or able to scavenge free radicals.

The standard method to remove the opaque nucleus of a lens to create a capsular bag for insertion of an artificial intraocular lens (IOL) is a phacoemulsification by a device which generates ultrasound oscillations. Especially the endothelial layer of orderly arranged cuboidal cells, which are located on the Descemet membrane at the anterior side of the cornea, can be affected by this iatrogenic procedure. The endothelial cell layer maintains corneal clarity by mediating a net flux of ions
from the stroma to the aqueous humor which keeps the stroma in a relatively dehydrated state. The density of these cells is for an elderly cataract patient app. 2600 cell/mm². Corneal endothelial cells are not able to divide and if their density decreases down to 500 cell/mm² there is a risk of corneal edema because the dehydrated state of the stroma and therefore the clarity cannot be maintained any more. It is one of the most serious complications which were noted frequently at the early phacoemulsification technology. The delicate endothelial cell layer can be damaged e.g. via generated hydroxyl radicals; and the biochemical and mechanical effects of the irrigation solution. The oxygen free radicals are the result of ultrasonic oscillations that induce acoustic cavitations which lead to dissociation of water vapor. It has been proposed that theses free radicals are a significant source of endothelial damage after cataract extraction by inducing apoptosis. The endothelium can be protected by an ophthalmic viscosurgical device composted of a viscoelastic polymer, e.g. hydroxypropyl methylcellulose (HPMC), chondroitin sulfate or hyaluronic acid (HA). However, the protection against free radicals can be significantly increased by the use of scavengers that are able to buffer the amount of oxygen-free radicals and therefore increase the protection performance of OVD in addition to the viscoelastic property, the suitable pH value and osmolality to reduce the loss of endothelial cells during cataract surgery.

Combinations of OVDs and scavenger molecules via a mixture or ionic bonding cannot safely prevent a dissociation of the scavenger molecules out of the OVD. However, diffusion into body fluids could cause unwanted local or systemic pharmacological effects by binding on a receptor target. The place of activity of a scavenger for tissue protection is the extracellular lumen of the anterior chamber. If for instance free diffusing membrane-permeable tempol is used as scavenger, it may permeate into adjacent cells.

To securely avoid these problems, a tight fixation to an OVD restricts the radical scavenging activity to the location where the free radicals are generated. Further, scavenger molecules with low water solubility can be used as an aggregation is prevented by the covalent attachment to the viscoelastic polymer, e.g. to the highly hydrophilic hyaluronic acid. Binding of a hydrophobic scavenger will decrease the hydrophilic property of the hyaluronic acid or any other viscoelastic polymer only negligibly.

The amount of scavenger molecules per viscoelastic polymer molecule can be adjusted as needed in order to get the appropriate rheological properties and optimal radical scavenge function.

Hyaluronic acid is a viscoelastic polysaccharide with repeating disaccharide units of glucuronosyl-β-1,3-N-acetylglucosamine linked by β-1,4-glycoside (glycosidic) bonds and has the general formula

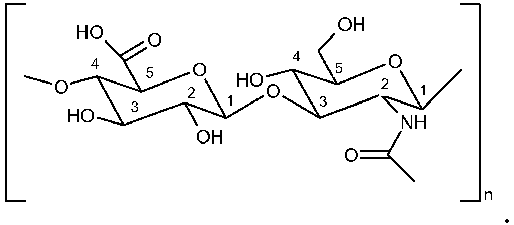

The carboxyl-groups or the primary and secondary hydroxyl-groups of hyaluronic acid can be used to bind scavenger molecules covalently to get an OVD with an additional epithelial cell protection by removal of free radicals which are generated by phacoemulsification of the lens.

In the simplest configuration, a preferably commercially available scavenger is used which has preferably already functional groups which can be linked selectively to the viscoelastic polymer in question. Alternatively, it is possible to modify the scavenger molecule and/or the viscoelastic polymer by introduction of adequate functional groups.

The amount of free radicals produced during phacoemulsification can be identified e.g. by electron spin resonance (ESR) spectroscopy. Scavenger molecules are able to react with those free radicals and form radicals that are thermodynamically more stable. For some scavenging molecules, e.g. the phenolic compound class of the flavones, this is enabled by delocalization of an impaired electron into the aromatic delocalized π-electron system. Therefore care should be taken not to disrupt the aromatic structure elements by the selected coupling reaction as this may impair the scavenging activity.

Flavones share a common structural element (2-phenyl-4H-chromen-4-one) with the general formula

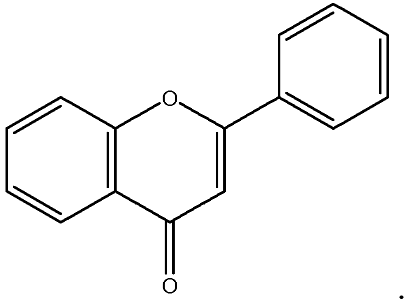

The commercially available 4'-amino-6-hydroxyflavone (2-(4-aminophenyl)-6-hydroxy-4H-chromen-4-one), having the formula 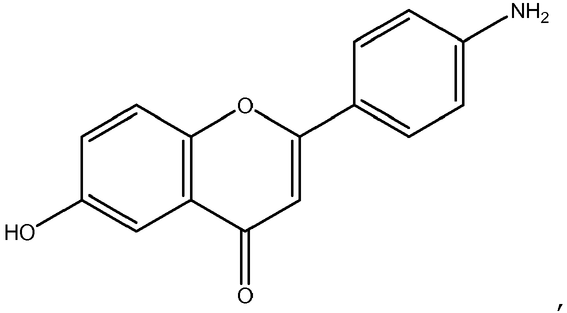 can be covalently bound to carboxyl-groups of hyaluronic acid (HA). A HA-flavone-conjugate can be obtained e.g. by the reaction of CNBr-activated hyaluronic acid with vicinal diols and 4'-amino-6-hydroxyflavone, resulting in the following structure:

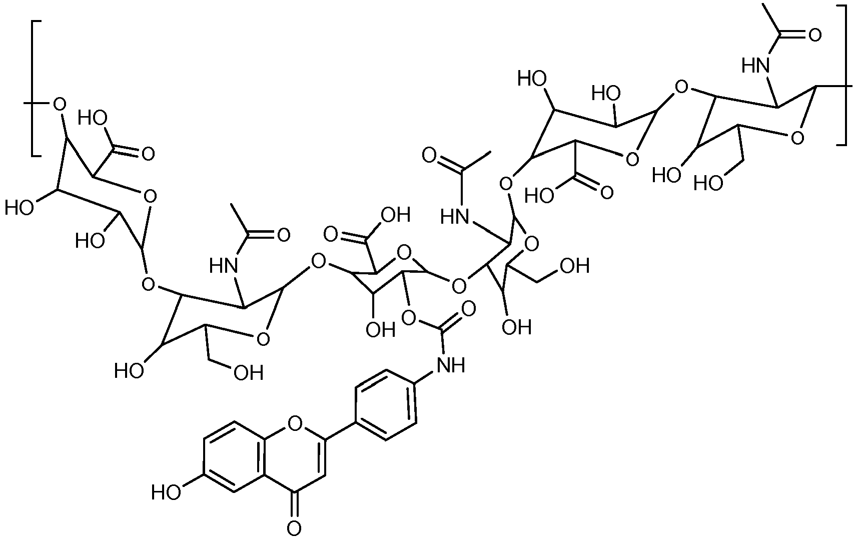

If phenolic compounds are used, for example hydroxyflavones like 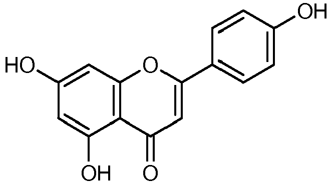 (apigenin), 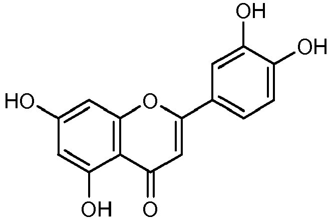 (luteolin), or 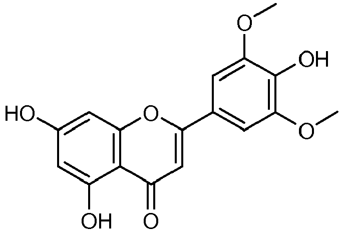 (chrysin),
their phenolic hydroxyl groups may be used to bind to the HA carboxyl-groups by an esterification reaction, for example via corresponding acid chlorides or acid anhydrates in the presence of potassium carbonate or pyridine according to the so-called Schotten-Baumann reaction. The resulting flavone-hyaluronic acid conjugate has the following structure: 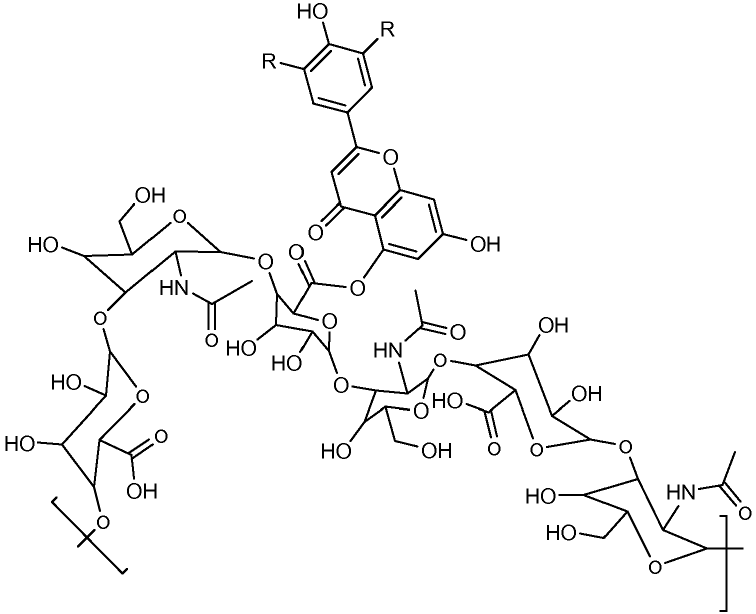 (with R= H, OH, OMe).

It is understood that various positional isomers can be formed by this reaction if the phenolic compound comprises a plurality of hydroxyl groups.

A scavenger function may further be realized by covalently binding alpha-tocopherol (vitamin E) and/or tempol to a viscoelastic polymer. A covalent binding to the highly hydrophilic hyaluronic acid allows to create a homogeneous OVD phase with free radical scavenging activity. However, the water solubility of the liposoluble alpha-tocopherol is below of 1 mg/l. Therefore, the application of a-tocopherol to an aqueous solution of hyaluronic acid is less effective when trying to covalently bind a-tocopherol. However, the coupling reaction does not necessarily have to take place in the same phase. The coupling reaction can also take place at the interface between two phases of immiscible solvents containing the scavenger and hyaluronic acid in the particular solvent phases. This is known as interfacial reaction in a two-phase system.

One possible method to prepare an ester-bond between a-tocopherol and HA under mild conditions includes the use of trifluoroacetic acid anhydride, resulting in the following HA-tocopherol-conjugate:

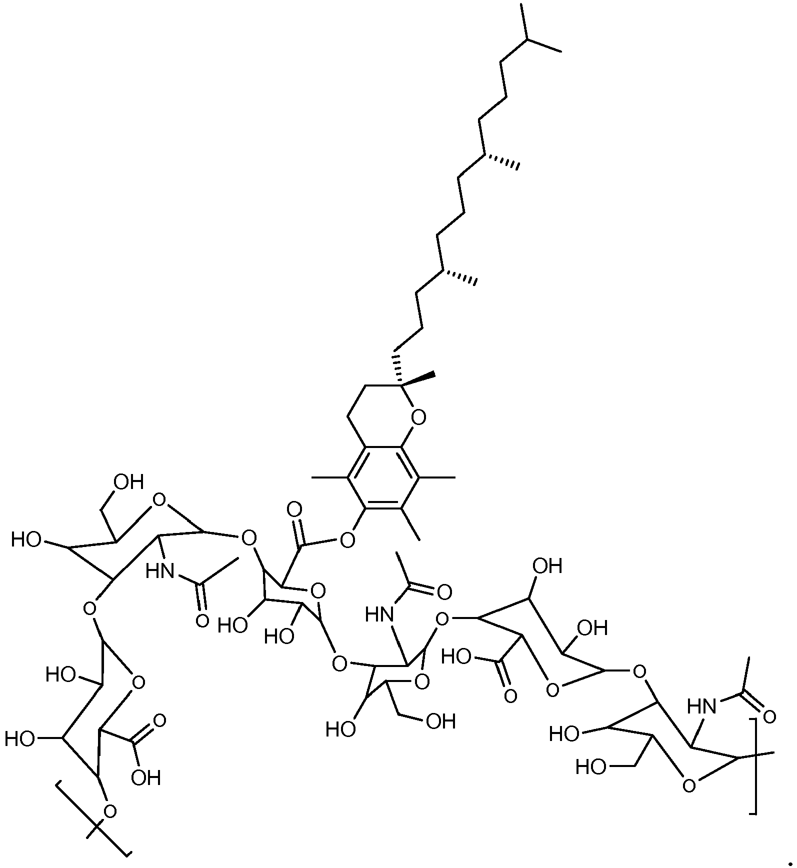

Tempol can be bound to the hyaluronic acid via the carboxyl groups by the mild Steglich esterification with dicydohexylcarbodiimid (DCC) and 4-(dimethylamino)-pyridin (DMAP) as catalyst according to the general reaction equation:

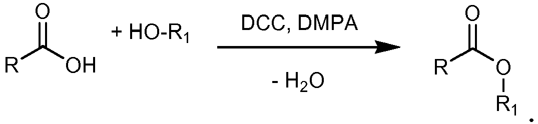

The resulting HA-Tempol-conjugate has the general formula:

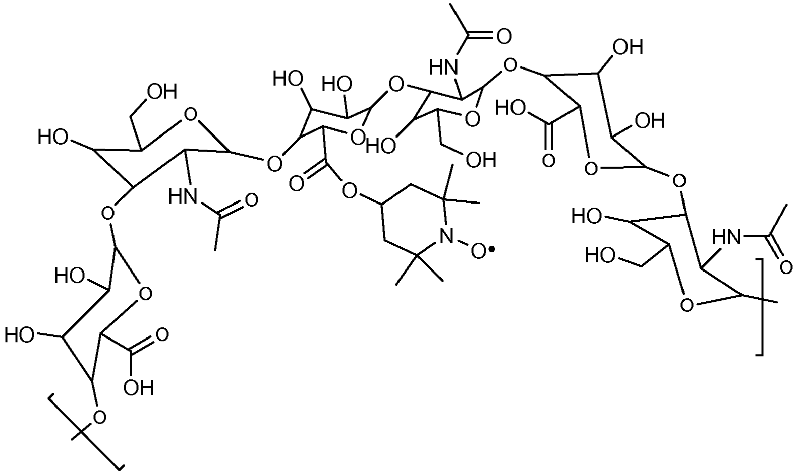

Alternatively or additionally to hyaluronic acid, basically, other viscoelastic polymers such as for example hydroxypropylmethylcellulose, chondroitin sulfate or mixtures thereof can also be used.

In the ophthalmic surgery, the OVD according to the invention can be used for filling up the vitreous body as well as for stabilizing the anterior chamber and for protection of the highly sensible endothelial cell layer of the cornea during surgery on the anterior eye portions, especially the surgery of the cataract. As the viscoelastic polymer, to which the dye/scavenger is covalently bound, for example hyaluronic acid is suited.

An example of the invention relates to OVDs comprising at least one viscoelastic polymer, wherein the at least one viscoelastic polymer is covalently bound to at least one scavenger, wherein the scavenger is a phenolic compound, and additionally is covalently bound to at least one dye. Suitable dyes that can be covalently bound to hyaluronic acid or another polysaccharide are based on aminoanthracenedione derivatives of the general formula (I): 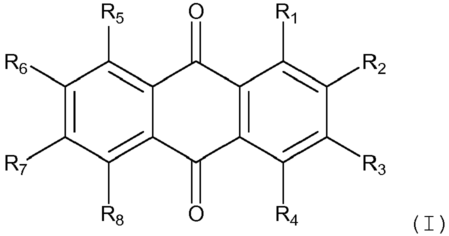 wherein in the general formula (I) at least one of the substituents R₁ to R₈ is an amino group, and the remaining substituents R₁ to R₈, which are different from the amino group, are selected from:
hydrogen, halogen, C₁-C₄-alkyl, C₁-C₄-alkoxy, C₁-C₄-alkoxy-C₁-C₄-alkyl, C₁-C₄-alkyl-C₁-C₄-alkoxy, C₁-C₄-alkoxy-C₁-C₄-alkoxy, aryloxy, acetamide, propionamide, butyramide, isobutyramide, sulphonate, (aminophenyl)-N-alkylacetamide, (aminophenylsulphonyl)alkyl sulphate, alkylbenzenesulphonamide, tri(alkyl)benzenamine, hydroxy, (alkylsulphonyl)benzenamine, and (alkenylsulphonyl)benzenamine, and/or
wherein at least two adjacent substituents Rₓ, Rₓ₊₁ with x=1 to 3 and/or 5 to 7 form a 3-, 4-, 5-, 6- or 7-membered homocyclic or heterocyclic, unsaturated or aromatic radical.

Alternatively or additionally a reactive dye from the group of ((nitrophenyl)diazenyl)benzenamine derivatives having the general formula (II) 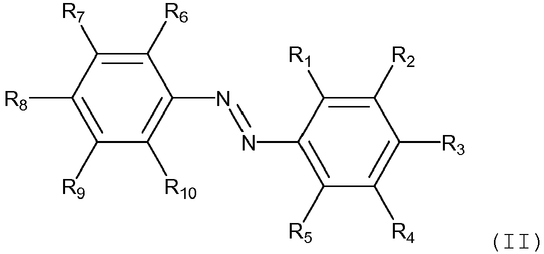 may be covalently bound to the polysaccharide, wherein in the general formula (II) at least one of the substituents R₁ to R₅ is an amino group and at least one of the substituents R₆ to R₁₀ is a nitro group, and wherein the remaining radicals R₁ to R₁₀, which are different from the amino group and the nitro group, are selected from:
hydrogen, halogen, C₁-C₄-alkyl, C₁-C₄-alkoxy, C₁-C₄-alkoxy-C₁-C₄-alkyl, C₁-C₄-alkyl-C₁-C₄-alkoxy, C₁-C₄-alkoxy-C₁-C₄-alkoxy, aryloxy, acetamide, propionamide, butyramide, isobutyramide, sulphonate, (aminophenyl)-N-alkylacetamide, (aminophenylsulphonyl)alkyl sulphate, alkylbenzenesulphonamide, tri(alkyl)benzenamine, hydroxy, (alkylsulphonyl)benzenamine, and (alkenylsulphonyl)benzenamine, and/or
wherein at least two adjacent substituents R_{y}, R_{y+1} with y=1 to 4 and/or 6 to 9 form the cyclic radical 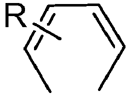
with:
   R=hydrogen, halogen, amino, hydroxyl, nitro, C₁-C₄-alkyl, C₁-C₄-alkoxy, C₁-C₄-alkoxy-C₁-C₄-alkyl, C₁-C₄-alkyl-C₁-C₄-alkoxy, and/or C₁-C₄-alkoxy-C₁-C₄-alkoxy.

Since both the dyes of the general formula (I) and the dyes of the general formula (II) have free amino groups, they can be bound to a polysaccharide having free keto, aldehyde and/or carboxyl groups without further derivatization or functionalization with the aid of the so-called Ugi reaction. The Ugi reaction is a 4 component condensation (U-4CC), which allows the synthesis of α-aminoacylamide derivatives from aldehydes, amines, carboxylic acids and isocyanides. Therein, the Ugi reaction proceeds according to the general reaction scheme:

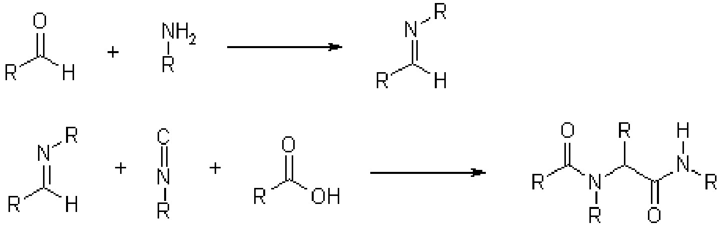

In the specific case, thus, the dyes of the general formulas (I) und (II) function as the amino component and the hyaluronic acid functions as the carboxylic acid component of the Ugi reaction. For example, acetaldehyde can be used as the aldehyde. For example, cyclohexanenitrile is suitable as the isocyanide. Generally, low molecular alcohols have proven as the solvent in the Ugi reaction.

Preferably, the reactants are prepared in high concentrations with cooling and react very fast, that is within a few minutes at room temperature. Lewis acids known per se can be employed in the conversion in particular of sterically demanding educts for catalysis.

In the case of hyaluronic acid, which has the general formula 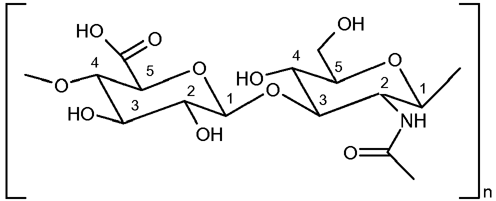 the dyes of the general formulas (I) and (II) thus can react with the free carboxyl groups of the hyaluronic acid with the aid of their amino groups in a reaction step and hereby be covalently bound to the hyaluronic acid. The carboxylic acids employed within the scope of an Ugi reaction can advantageously be diversely varied such that other polysaccharides with free carboxyl groups can also be used without previous functionalization within the scope of an Ugi reaction. Polysaccharides without carboxyl groups can be subjected to a preceding derivatization or functionalization for later use in the Ugi reaction. Conversely, polysaccharides with free amino groups can of course also be reacted with dyes having free carboxyl groups.

An advantage of the dyes of the general formulas (I) and (II) is in their basically very high extinction coefficient in the visible range of the light spectrum (about 400 nm to about 800 nm). Furthermore, the color of the dyes of the general formulas (I) and (II) can be simply varied in a wide color range by use of corresponding substituents and/or position isomers, whereby the ophthalmologic composition can be simply adapted to different purposes of use.

In the following table 1, various embodiments for dyes of the general formula (I) are specified.

**Table 1: Embodiments for dyes of the general formula (I)**

| | |
|---|---|
| Example 1 | 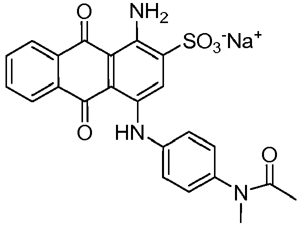 |
| Name and Synonyms | sodium 1-amino-4-(4-(N-methyl-acetamido)phenylamino)-9,10-dioxo-9,10-dihydroanthracene-2-sulfonate; Acid Blue 41; CAS 2666-17-3 |
| Example 2 | 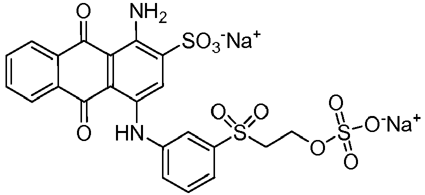 |
| Name and Synonyms | sodium 2-(3-(4-amino-9,10-dioxo-3-sulfonato-9,10-dihydroanthracen-1-ylamino)phenylsulfonyl)ethyl sulphate; Reactive Blue 19 (Remazol Brilliant Blue R); CAS 2580-78-1 |
| Example 3 | 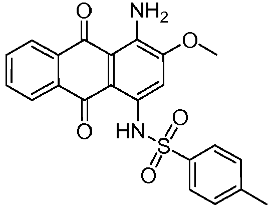 |
| Name and Synonyms | N-(4-amino-3-methoxy-9,10-dioxo-9,10-dihydroanthracen-1-yl)-4-methylbenzenesulfonamide; Disperse Red 86; CAS 81-68-5 |
| Example 4 | 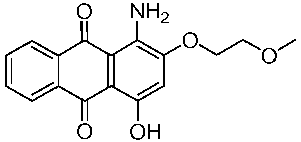 |
| Name and Synonyms | 1-amino-4-hydroxy-2-(2-methoxyethoxy)anthracene-9,10-dione; Disperse Red 59; CAS 17869-10-2 |
| Example 5 | 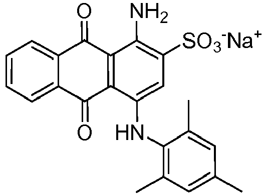 |
| Name and Synonyms | sodium 1-amino-4-(mesitylamino)-9,10-dioxo-9,10-dihydroanthracene-2-sulfonate; Acid Blue 129; CAS 6397-02-0 |
| Example 6 | 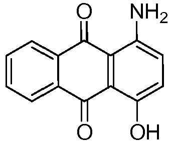 |
| Name and Synonyms | 1-amino-4-hydroxyanthracene-9,10-dione; Disperse Red 15; CAS 116-85-8 |
| Example 7 | 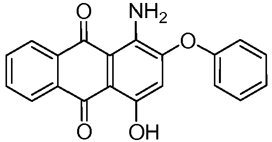 |
| Synonyms | 1-amino-4-hydroxy-2-phenoxyanthracene-9,10-dione; Disperse Red 60; CAS 17418-58-5 |
| Example 8 | 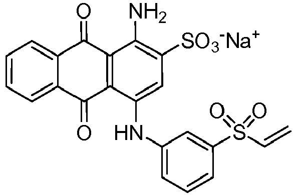 |
| Name and Synonyms | sodium 1-amino-9,10-dioxo-4-(3-(vinylsulfonyl)phenylamino)-9,10-dihydroanthracene-2-sulfonate; UniBlue A Sodium Salt; CAS 14541-90-3 |

In the following table 2, various embodiments for dyes of the general formula (II) are specified.

**Table 2: Embodiments for dyes of the general formula (II)**

| | |
|---|---|
| Example 1 | 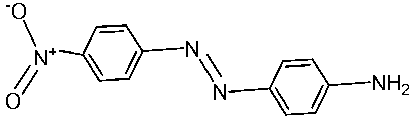 |
| Name and Synonyms | (E)-4-((4-nitrophenyl)diazenyl)benzenamine; Disperse Orange 3; CAS 730-40-5 |
| Example 2 | 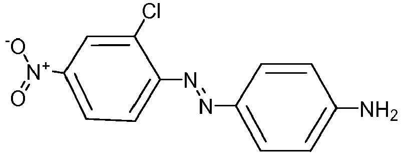 |
| Name and Synonyms | (E)-4-((2-chloro-4-nitrophenyl)diazenyl)benzenamine; 4-[(2-chloro-4-nitrophenyl)azo]aniline; CAS 52735-98-5 |
| Example 3 | 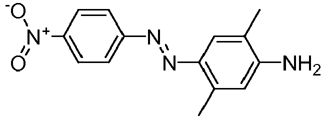 |
| Name and Synonyms | (E)-2,5-dimethyl-4-((4-nitrophenyl)diazenyl)benzenamine; 4-[(4-nitrophenyl)azo]-2,5-xylidine; CAS 6492-50-8 |
| Example 4 | 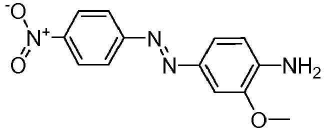 |
| Name and Synonyms | (E)-2-methoxy-4-((4-nitrophenyl)diazenyl)benzenamine; 4-[(4-nitrophenyl)azo]-o-anisidine; CAS 101-52-0 |
| Example 5 | 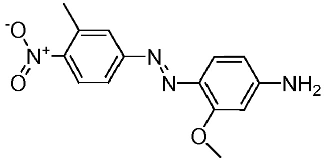 |
| Name and Synonyms | (E)-3-methoxy-4-((3-methyl-4-nitrophenyl)diazenyl)benzenamine; 4-Amino-2-methoxy-3'-methyl-4-nitroazobenzene; CAS 144829-57-2 |
| Example 6 | 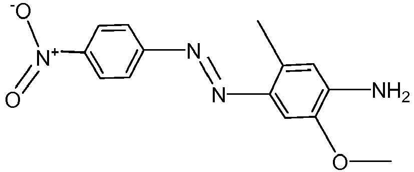 |
| Name and Synonyms | (E)-2-methoxy-5-methyl-4-((4-nitrophenyl)diazenyl)benzenamine; Disperse Red 31; CAS 2475-43-6 |
| Example 7 | 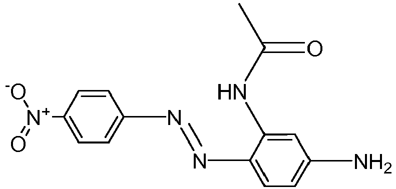 |
| Name and Synonyms | N-[5-amino-2-[(p-nitrophenyl)azo]phenyl]acetamide; CAS 26311-09-1 |
| Example 8 | 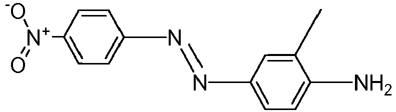 |
| Name and Synonyms | (E)-2-methyl-4-((4-nitrophenyl)diazenyl)benzenamine; 4-[(4-nitrophenyl)azo]-o-toluidine; CAS 84255-13-0 |
| Example 9 | 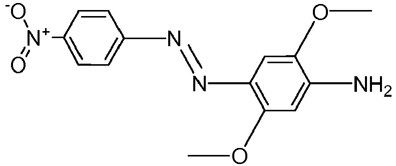 |
| Name and Synonyms | (E)-2,5-dimethoxy-4-((4-nitrophenyl)diazenyl)benzenamine; 2,5-dimethoxy-4-(4-nitrophenylazo)aniline; CAS 6358-51-6 |
| Example 10 | 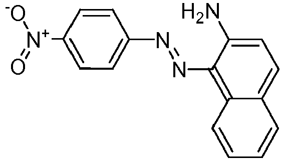 |
| Name and Synonyms | (E)-1-((4-nitrophenyl)diazenyl)naphthalene-2-amine; 1-(4'-Nitrophenylazo)-2-naphthylamine; Solvent Red 5; CAS 3025-77-2 |
| Example 11 | 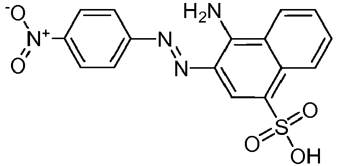 |
| Name and Synonyms | (E)-4-amino-3-((4-nitrophenyl)diazenyl)naphthalene-1-sulphonic acid; Acid Red 74; CAS 6300-18-1 |

However, it is to be emphasized that besides the Ugi reaction, basically, other chemical reaction types can also be used for covalently binding the concerned dye and/or the concerned scavenger compound to the concerned viscoelastic polymer. For example, dyes with a free alcohol group- like phenolic compounds - can be bound to hyaluronic acid by an esterification reaction.

Labeling of hyaluronic acid (HA) can for example also be obtained by the reaction of CNBr-activated HA at vicinal diols and a dye comprising a reactive amine group according to the following reaction scheme: 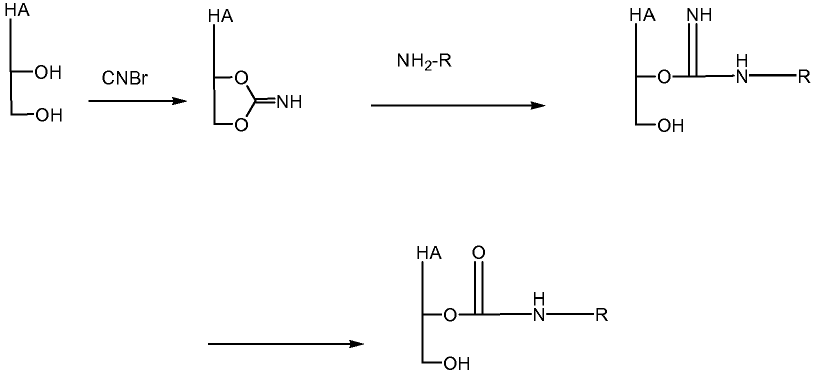 wherein HA denotes hyaluronic acid and NH₂-R denotes the reactive dye comprising an amine group. The reaction may also involve the hydroxyl groups in two hyaluronic acid molecules.

It is also possible to use a phenolic compound and/or a further scavenger molecule and/or a dye comprising an isothiocyanate group (denoted by SCN-R), which targets mostly the primary hydroxyl groups of HA or other viscoelastic polysaccharides:

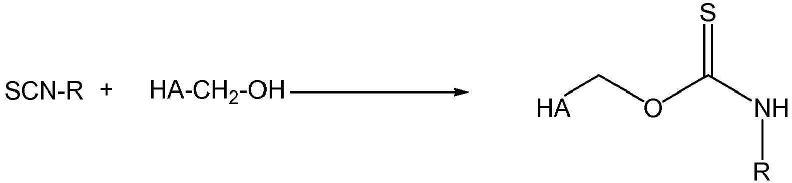

The reaction is conducted at 37°C overnight. The resulting color labeled polysaccharide is stable for over a month.

A further type of reaction for covalently binding a dye to hyaluronic acid includes activation of a hyaluronic acid derivative with N-hydroxysuccinimide coupled diphenylphosphoric acid. The resulting adduct is reacted with a dye comprising a reactive hydrazide group (denoted by [R]CC(NN)=O) according to the following general reaction scheme:

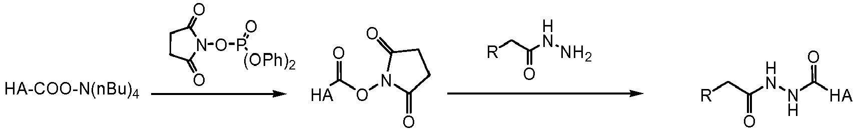

Another method to chemically bond a reactive dye to hyaluronic acid (HA) or other polysaccharides comprising carboxylic groups is depicted in the following reaction scheme:

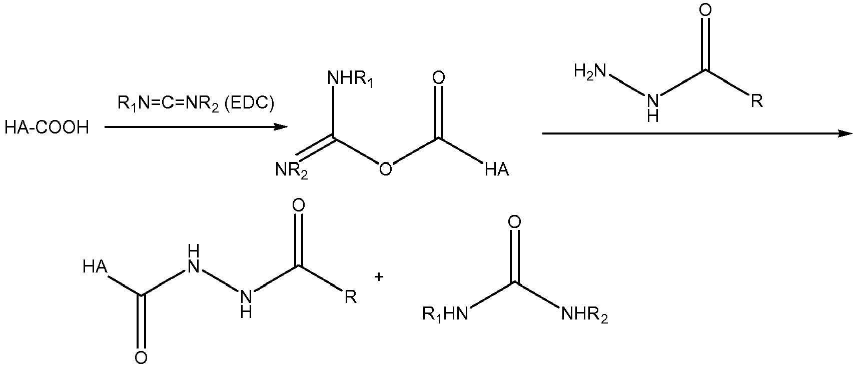

The reaction employs a dye comprising a hydrazide group. The first step is to form an O-acylurea of HA by reaction of HA with 1-ethyl-3-(3-dimethylaminopropyl)carbodiimide (EDC) having the formula

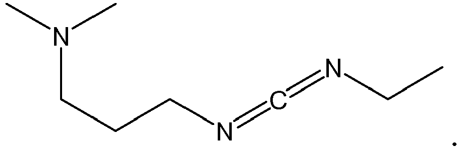

The resulting intermediate is then reacted with the reactive dye to form the color labeled HA.

Another possibility is the reaction between the carboxylic groups of HA and dyes comprising a carbodiimide group in the presence or absence of primary amines at pH 4.5 according to the general reaction scheme:

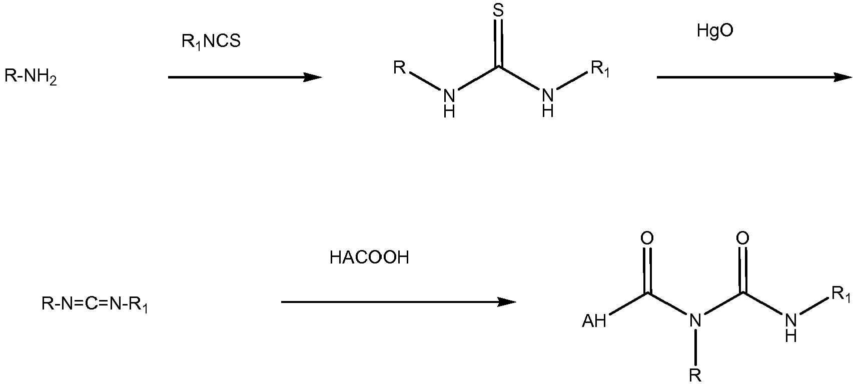

The reaction of carbodiimides proceeds rapidly at room temperature. The final group is an N-acylurea or O-acylurea. The carbodiimides are synthesized in a 2-step reaction: the reaction of a dye comprising a primary amine and isothiocyanate to form a thiourea and the reaction of said thiourea and HgO to produce the reactive dye comprising a carbodiimide group.

HA esters can also be produced by a nucleophilic reaction of a reactive dye comprising a halide with a quaternary salt of hyaluronic acid:

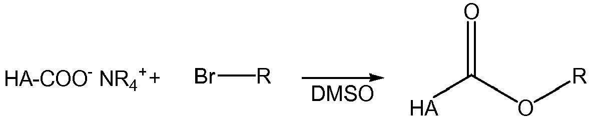

Another way to molecularly label the viscoelastic polysaccharide is given by the reaction of HA with diepoxies:

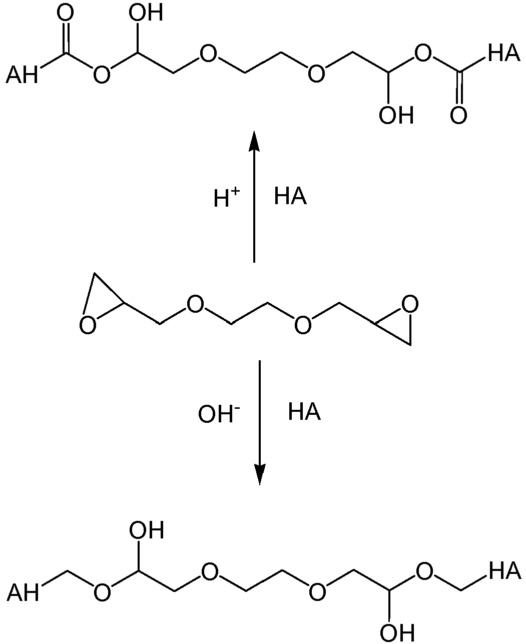

In acidic conditions, the ester of HA is formed by linking the epoxy to HA at the acidic position. In basic conditions, the ether is produced by linking the epoxy to HA at the primary alcohol position.

Another way to functionalize HA at the primary alcohol position is to use divinylsulfone:

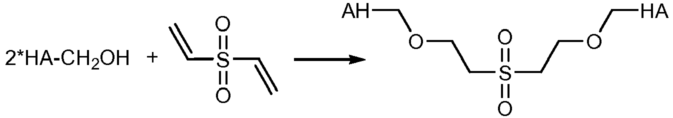

Alternatively, it can be provided herein that a dye with a free hydroxy group is used and bound to the hyaluronic acid with the aid of a linking agent.

Alternatively or additionally to hyaluronic acid, basically, other viscoelastic polymers, in particular polysaccharides, such as for example hydroxypropylmethylcellulose, chondroitin sulfate or mixtures thereof can also be used.

An OVD (or ophthalmologic composition) comprising an aqueous solution of a viscoelastic polysaccharide which is covalently bound to a scavenger compound and may further contain one or several dyes of the general formulas (I) and/or (II) may be used in a phacoemulsification method. During the phacoemulsification, the anterior chamber of the eye is filled with the ophthalmologic composition comprising the colored viscoelastic solution. The viscoelastic OVD is used as a surgical aid to protect intraocular tissues, for example the corneal endothelium, during the phacoemulsification, as a space maintainer to maintain the anterior chamber of the eye, and to facilitate intraocular maneuvers, for example to make a controlled capsulorhexis. Because the scavenger and the dye are covalently bound to the viscoelastic polymer, the scavenger and the dye do not diffuse out of the solution and therefore do not negatively affect or stain the surrounding tissues.

After the capsulorhexis the colored OVD may be completely removed from the anterior chamber as the surgeon can visualize even very small traces of the colored viscoelastic polymer.

## Claims

1. An ophthalmic viscoelastic device, comprising at least one viscoelastic polymer, wherein the at least one viscoelastic polymer is covalently bound to at least one phenolic compound,
**characterized in that**
the at least one viscoelastic polymer is covalently bound to at least one dye.

2. The ophthalmic viscoelastic device according to claim 1,
**characterized in that**
the at least one phenolic compound is bound directly and/or via a spacer to the viscoelastic polymer.

3. The ophthalmic viscoelastic device according to claim 1 or 2,
**characterized in that**
the viscoelastic polymer comprises a polysaccharide.

4. The ophthalmic viscoelastic device according to claim 3,
**characterized in that**
the polysaccharide is cellulose, a cellulose ether with methyl and/or ethyl and/or propyl groups, in particular hydroxypropyl methylcellulose, hydroxyethyl methylcellulose and/or methylcellulose, a glycosaminoglycan, in particular hyaluronic acid, chondroitin sulphate, dermatan sulphate, heparin, heparan sulphate, keratan sulphate, alginic acid, polymannuronic acid, polyguluronic acid, polyglucuronic acid, amylose, amylopectin, callose, chitosan, polygalactomannan, dextran, xanthan and/or a mixture thereof.

5. The ophthalmic viscoelastic device according to any one of claims 1 to 4,
**characterized in that**
the dye comprises a phenolic compound and/or a reactive dye from the group of modified and/or unmodified aminoanthracenedione and/or of modified and/or unmodified nitrophenyldiazenylbenzenamine.

6. The ophthalmic viscoelastic device according to any one of claims 1 to 5,
**characterized in that**
the at least one phenolic compound is selected from a group of modified and/or unmodified hydroxycinnamic acids, phenylpropenes, cumarins, hydroxycumarines, isocoumarins, chromones, hydroxybenzoic acids, in particular salicylic acid and acetylsalicylic acid, tocopheroles, tocotrienols, propofol, phenolic acids, phenolic aldehydes, N-(2,3-dihydro-7-hydroxy-2,2,4,6-tetramethyl-1H-inden-1-yl) -4-(3-methoxyphenyl)-1-piperazineacetamide, butylhydroxyanisole, butylhydroxytoluene, galvinoxyl, benzoquinones, acetophenones, tyrosine derivatives, phenylacetic acids, naphthoquinones, xanthonoids, stilbenoids, in particular resveratrol, anthraquinones, flavonols, dihydroflavonols, tannins, pseudo tannins, anthocyanins, anthocyanidins, flavanol monomers, in particular catechins, flavanol polymers, in particular proanthocyanidins, flavanones, flavones, chalconoids, isoflavonoids, neoflavonoids, lignans, neolignans, biflavonoids, catechol melanins, flavolans, theaflavins, and thearubigins.

7. The ophthalmic viscoelastic device according to any one of claims 1 to 6,
**characterized in that**
the at least one phenolic compound is glycosylated.

8. The ophthalmic viscoelastic device according to any one of claims 1 to 7,
**characterized in that**
the zero-shear viscosity of the ophthalmic viscoelastic device is between 20,000 and 8,000,000 mPa.s, preferably between 50,000 and 500,000 mPa.s.

9. The ophthalmic viscoelastic device according to any one of claims 1 to 8,
**characterized in that**
it is a water-based ophthalmologic solution to protect intraocular tissues in eye surgery, wherein the aqueous solution contains the viscoelastic polymer, to which the at least one phenolic compound is covalently bound.

10. A method for producing an ophthalmic viscoelastic device comprising at least one viscoelastic polymer,
comprising the steps of
- covalently bonding the at least one viscoelastic polymer to at least one phenolic compound;
- covalently bonding the at least one viscoelastic polymer to at least one dye; and
- using the at least one viscoelastic polymer as an ingredient of the ophthalmic viscoelastic device.

11. The method according to claim 10,
**characterized in that**
for producing the ophthalmic viscoelastic device the viscoelastic polymer, which is covalently bound to the at least one phenolic compound, and/or a physiologically acceptable salt thereof is dissolved and/or dispersed in an amount of between 0.05 percent by weight and 5 percent by weight possibly together with a buffer system and/or with further agents and/or excipients in a protic solvent, in particular in water.

12. The method according to claim 10 or 11,
**characterized in that**
in order to bond the at least one phenolic compound, the viscoelastic polymer and/or the at least one phenolic compound is functionalized and covalently bound via the added functional group.

13. The method according to any one of claims 10 to 12,
**characterized in that**
that the at least one phenolic compound is covalently bound to a carboxylic group and/or to a primary hydroxyl group of the viscoelastic polymer.

14. The method according to any one of claims 10 to 13,
**characterized in that**
a viscoelastic polymer having a molecular weight of between 500,000 u and 5,000,000 u, preferably between 800,000 u and 2,500,000 u, is covalently bound to the at least one phenolic compound.

## Patentansprüche

1. Ophthalmische viskoelastische Vorrichtung, die wenigstens ein viskoelastisches Polymer umfasst, wobei das wenigstens eine viskoelastische Polymer kovalent an wenigstens eine Phenolverbindung gebunden ist,
**dadurch gekennzeichnet, dass**
das wenigstens eine viskoelastische Polymer kovalent an wenigstens einen Farbstoff gebunden ist.

2. Ophthalmische viskoelastische Vorrichtung gemäß Anspruch 1,
**dadurch gekennzeichnet, dass**
die wenigstens eine Phenolverbindung direkt und/oder über ein Zwischenstück an das viskoelastische Polymer gebunden ist.

3. Ophthalmische viskoelastische Vorrichtung gemäß Anspruch 1 oder 2,
**dadurch gekennzeichnet, dass**
das viskoelastische Polymer ein Polysaccharid umfasst.

4. Ophthalmische viskoelastische Vorrichtung gemäß Anspruch 3,
**dadurch gekennzeichnet, dass**
das Polysaccharid Cellulose, ein Celluloseether mit Methyl- und/oder Ethyl- und/oder Propylgruppen, insbesondere Hydroxypropylmethylcellulose, Hydroxyethylmethylcellulose und/oder Methylcellulose, ein Glycosaminoglycan, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopectin, Callose, Chitosan, Polygalactomannan, Dextran, Xanthan und/oder ein Gemisch davon ist.

5. Ophthalmische viskoelastische Vorrichtung gemäß einem der Ansprüche 1 bis 4,
**dadurch gekennzeichnet, dass**
der Farbstoff eine Phenolverbindung und/oder einen reaktiven Farbstoff aus der Gruppe von modifiziertem und/oder unmodifiziertem Aminoanthracendion und/oder von modifizierten und/oder unmodifiziertem Nitrophenyldiazenylbenzolamin umfasst.

6. Ophthalmische viskoelastische Vorrichtung gemäß einem der Ansprüche 1 bis 5,
**dadurch gekennzeichnet, dass**
die wenigstens eine Phenolverbindung ausgewählt ist aus der Gruppe von modifizierten und/oder unmodifizierten Hydroxyzimtsäuren, Phenylpropenen, Cumarinen, Hydroxycumarinen, Isocumarinen, Chromonen, Hydroxybenzoesäuren, insbesondere Salicylsäure und Acetylsalicylsäure, Tocopherolen, Tocotrienolen, Propofol, Phenolsäuren, Phenolaldehyden, N-(2,3-Dihydro-7-hydroxy-2,2,4,6-tetramethyl-1H-inden-1-yl)-4-(3-methoxyphenyl)-1-piperazinacetamid, Butylhydroxyanisol, Butylhydroxytoluol, Galvinoxyl, Benzochinonen, Acetophenonen, Tyrosinderivaten, Phenylessigsäuren, Naphthochinonen, Xanthonoiden, Stilbenoiden, insbesondere Resveratrol, Anthrachinonen, Flavonolen, Dihydroflavonolen, Tanninen, Pseudotanninen, Anthocyaninen, Anthocyanidinen, Flavanolmonomeren, insbesondere Catechinen, Flavanolpolymeren, insbesondere Proanthocyanidinen, Flavanonen, Flavonen, Chalconoiden, Isoflavonoiden, Neoflavonoiden, Lignanen, Neolignanen, Biflavonoiden, Catecholmelaninen, Flavolanen, Theaflavinen und Thearubiginen.

7. Ophthalmische viskoelastische Vorrichtung gemäß einem der Ansprüche 1 bis 6,
**dadurch gekennzeichnet, dass**
die wenigstens eine Phenolverbindung glycosyliert ist.

8. Ophthalmische viskoelastische Vorrichtung gemäß einem der Ansprüche 1 bis 7,
**dadurch gekennzeichnet, dass**
die Nullscherungsviskosität der ophthalmischen viskoelastischen Vorrichtung zwischen 20.000 und 8.000.000 mPa.s, vorzugsweise zwischen 50.000 und 500.000 mPa.s, beträgt.

9. Ophthalmische viskoelastische Vorrichtung gemäß einem der Ansprüche 1 bis 8,
**dadurch gekennzeichnet, dass**
sie eine ophthalmologische Lösung auf Wasserbasis zum Schützen von intraokularen Geweben bei der Augenchirurgie ist, wobei die wässrige Lösung das viskoelastische Polymer enthält, an das die wenigstens eine Phenolverbindung kovalent gebunden ist.

10. Verfahren zum Herstellen einer ophthalmischen viskoelastischen Vorrichtung, die wenigstens ein viskoelastisches Polymer umfasst,
umfassend die Schritte
- kovalentes Binden des wenigstens einen viskoelastischen Polymers an wenigstens eine Phenolverbindung;
- kovalentes Binden des wenigstens einen viskoelastischen Polymers an wenigstens einen Farbstoff; und
- Verwenden des wenigstens einen viskoelastischen Polymers als Bestandteil der ophthalmischen viskoelastischen Vorrichtung.

11. Verfahren gemäß Anspruch 10,
**dadurch gekennzeichnet, dass**
zum Herstellen der ophthalmischen viskoelastischen Vorrichtung das viskoelastische Polymer, das kovalent an die wenigstens eine Phenolverbindung gebunden ist, und/oder ein physiologisch annehmbares Salz davon in einer Menge von zwischen 0,05 Gewichtsprozent und 5 Gewichtsprozent, gegebenenfalls zusammen mit einem Puffersystem und/oder mit weiteren Agenzien und/oder Hilfsstoffen, in einen protischen Lösungsmittel, insbesondere in Wasser, gelöst und/oder dispergiert wird.

12. Verfahren gemäß Anspruch 10 oder 11,
**dadurch gekennzeichnet, dass**
zum Binden der wenigstens einen Phenolverbindung das viskoelastische Polymer und/oder die wenigstens eine Phenolverbindung funktionalisiert und über die angefügte funktionelle Gruppe kovalent gebunden wird.

13. Verfahren gemäß einem der Ansprüche 10 bis 12,
**dadurch gekennzeichnet, dass**
die wenigstens eine Phenolverbindung kovalent an eine Carboxygruppe und/oder an eine primäre Hydroxygruppe des viskoelastischen Polymers gebunden wird.

14. Verfahren gemäß einem der Ansprüche 10 bis 13,
**dadurch gekennzeichnet, dass**
ein viskoelastisches Polymer mit einem Molekulargewicht von zwischen 500.000 u und 5.000.000 u, vorzugsweise zwischen 800.000 u und 2.500.000 u, kovalent an die wenigstens eine Phenolverbindung gebunden wird.

## Revendications

1. Dispositif ophtalmique viscoélastique, comprenant au moins un polymère viscoélastique, dans lequel ledit au moins un polymère viscoélastique est lié de façon covalente à au moins un composé phénolique,
**caractérisé en ce que**
ledit au moins un polymère viscoélastique est lié de façon covalente à au moins un colorant.

2. Dispositif ophtalmique viscoélastique selon la revendication 1,
**caractérisé en ce que**
ledit au moins un composé phénolique est lié directement et/ou par l'intermédiaire d'un espaceur au polymère viscoélastique.

3. Dispositif ophtalmique viscoélastique selon la revendication 1 ou 2,
**caractérisé en ce que**
le polymère viscoélastique comprend un polysaccharide.

4. Dispositif ophtalmique viscoélastique selon la revendication 3,
**caractérisé en ce que**
le polysaccharide est la cellulose, un éther de cellulose comportant des groupes méthyle et/ou éthyle et/ou propyle, en particulier l'hydroxypropylméthylcellulose, l'hydroxyéthylméthylcellulose et/ou la méthylcellulose, un glycosaminoglycane, en particulier l'acide hyaluronique, le sulfate de chondroïtine, le sulfate de dermatane, l'héparine, le sulfate d'héparane, le sulfate de kératane, l'acide alginique, l'acide polymannuronique, l'acide polyguluronique, l'acide polyglucuronique, l'amylose, l'amylopectine, le callose, le chitosane, le polygalactomannane, le dextrane, la gomme xanthane et/ou un mélange de ceux-ci.

5. Dispositif ophtalmique viscoélastique selon l'une quelconque des revendications 1 à 4,
**caractérisé en ce que**
le colorant comprend un composé phénolique et/ou un colorant réactif provenant du groupe constitué de l'aminoanthracènedione modifiée et/ou non modifiée et/ou de la nitrophényldiazénylbenzènamine modifiée et/ou non modifiée.

6. Dispositif ophtalmique viscoélastique selon l'une quelconque des revendications 1 à 5,
**caractérisé en ce que**
ledit au moins un composé phénolique est sélectionné dans un groupe constitué des acides hydroxycinnamiques modifiés et/ou non modifiés, des phénylpropènes, des coumarines, des hydroxycoumarines, des isocoumarines, des chromones, des acides hydroxybenzoïques, en particulier l'acide salicylique et l'acide acétylsalicylique, des tocophérols, des tocotriénols, du propofol, des acides phénoliques, des aldéhydes phénoliques, du N-(2,3-dihydro-7-hydroxy-2,2,4,6-tétraméthyl-1H-indén-1-yl)-4-(3-méthoxyphényl)-1-pipérazineacétamide, du butylhydroxyanisole, du butylhydroxytoluène, du galvinoxyle, des benzoquinones, des acétophénones, des dérivés de tyrosine, des acides phénylacétiques, des naphtoquinones, des xanthonoïdes, des stilbénoïdes, en particulier le resvératrol, des anthraquinones, des flavonols, des dihydroflavonols, des tanins, des pseudo-tanins, des anthocyanines, des anthocyanidines, des monomères de flavanol, en particulier les catéchines, des polymères de flavanol, en particulier les proanthocyanidines, des flavanones, des flavones, des chalconoïdes, des isoflavonoïdes, des néoflavonoïdes, des lignanes, des néolignanes, des biflavonoïdes, des catécholmélanines, des flavolanes, des théaflavines et des théarubigines.

7. Dispositif ophtalmique viscoélastique selon l'une quelconque des revendications 1 à 6,
**caractérisé en ce que**
ledit au moins un composé phénolique est glycosylé.

8. Dispositif ophtalmique viscoélastique selon l'une quelconque des revendications 1 à 7,
**caractérisé en ce que**
la viscosité à un cisaillement nul du dispositif ophtalmique est de 20 000 à 8 000 000 MPa•s, préférablement de 50 000 à 500 000 MPa•s.

9. Dispositif ophtalmique viscoélastique selon l'une quelconque des revendications 1 à 8,
**caractérisé en ce que**
il s'agit d'une solution aqueuse ophtalmologique conçue pour protéger les tissus intraoculaires durant la chirurgie oculaire, la solution aqueuse contenant le polymère viscoélastique, auquel ledit au moins un composé phénolique est lié de façon covalente.

10. Procédé de fabrication d'un dispositif ophtalmique viscoélastique comprenant au moins un polymère viscoélastique,
comprenant les étapes qui consistent à
- lier de façon covalente ledit au moins un polymère viscoélastique à au moins un composé phénolique ;
- lier de façon covalente ledit au moins un polymère viscoélastique à au moins un colorant ; et
- utiliser ledit au moins un polymère viscoélastique comme constituant du dispositif ophtalmique viscoélastique.

11. Procédé selon la revendication 10,
**caractérisé en ce que**
pour la fabrication du dispositif ophtalmique viscoélastique, le polymère viscoélastique, qui est lié de façon covalente audit au moins un composé phénolique, et/ou un sel physiologiquement acceptable de celui-ci, est dissous et/ou dispersé dans une quantité de 0,05 % en poids à 5 % en poids, éventuellement conjointement avec un système tampon et/ou avec des agents et/ou excipients supplémentaires dans un solvant protique, en particulier dans l'eau.

12. Procédé selon la revendication 10 ou 11,
**caractérisé en ce que**
afin de lier ledit au moins un composé phénolique, le polymère viscoélastique et/ou ledit au moins un composé phénolique sont fonctionnalisés et liés de façon covalente par l'intermédiaire du groupe fonctionnel ajouté.

13. Procédé selon l'une quelconque des revendications 10 à 12,
**caractérisé en ce que**
ledit au moins un composé phénolique est lié de façon covalente à un groupe carboxylique et/ou à un groupe hydroxyle primaire du polymère viscoélastique.

14. Procédé selon l'une quelconque des revendications 10 à 13,
**caractérisé en ce que**
un polymère viscoélastique ayant une masse moléculaire de 500 000 u à 5 000 000 u, préférablement de 800 000 u à 2 500 000 u, est lié de façon covalente audit au moins un composé phénolique.
